Europäisches Patentamt

European Patent Office

Office européen des brevets

(19)

(11) Publication number: **0 116 626**
**B1**

(12)

# EUROPEAN PATENT SPECIFICATION

(45) Date of publication of patent specification: **12.10.88**

(51) Int. Cl.⁴: **B 04 B 15/12, A 61 M 1/36**

(21) Application number: **83902860.2**

(22) Date of filing: **17.08.83**

(86) International application number:
**PCT/US83/01266**

(87) International publication number:
**WO 84/00905 15.03.84 Gazette 84/07**

(54) BLOOD COMPONENT COLLECTION SYSTEM AND METHOD.

<table>
<tr><td>

(30) Priority: **24.08.82 US 411058**

(43) Date of publication of application:
**29.08.84 Bulletin 84/35**

(45) Publication of the grant of the patent:
**12.10.88 Bulletin 88/41**

(84) Designated Contracting States:
**BE CH DE FR GB LI SE**

(56) References cited:
**EP-A-0 044 694**
**FR-A-2 406 449**
**US-A-2 876 769**
**US-A-3 228 876**
**US-A-3 655 123**
**US-A-3 870 042**
**US-A-4 187 979**
**US-A-4 215 688**
**US-A-4 267 269**
**US-A-4 280 497**
**US-A-4 286 597**
**US-A-4 332 122**
**US-A-4 381 775**

</td><td>

(73) Proprietor: **BAXTER INTERNATIONAL INC.**
**One Baxter Parkway**
**Deerfield, IL 60015 (US)**

(72) Inventor: **KRUGER, Robert, J.**
**1225 S. Patton**
**Arlington Heights, IL 60005 (US)**
Inventor: **BROWN, Richard, I.**
**2335 Peachtree Lane**
**Northbrook, IL 60062 (US)**

(74) Representative: **MacGregor, Gordon et al**
**ERIC POTTER & CLARKSON 14 Oxford Street**
**Nottingham, NG1 5BP (GB)**

</td></tr>
</table>

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a system for the collection and separation of whole blood into its therapeutic components according to the preamble of claim 1, and a method therefor according to the preamble of claim 6.

At the present time, over 12 million units of whole blood are collected from volunteer donors in the United States each year. Because of the advent of blood component therapy, approximately 60% to 80% of the whole blood collected today is not itself stored and used for transfusion. Instead, the whole blood is first separated into its clinically proven components, which are themselves individually stored and used to treat a multiplicity of specific conditions and diseased states.

The clinically proven components of whole blood include red blood cells, which can be used to treat chronic anemia; platelet-poor plasma, from which Clotting Factor VIII-rich cryoprecipitate can be obtained for the treatment of hemophilia; and concentrations of platelets, which can be used to control thrombocytopenic bleeding.

The present medical consensus is that care of a patient is improved by providing only the therapeutic components of whole blood which are required to treat the specific disease. The demand for therapeutic components of whole blood is thus ever-increasing. Likewise, the demand for safe and effective systems and methods for collecting, separating, and storing the therapeutic components of whole blood grows accordingly.

One desirable feature for a blood collection and sepaction system and method is the capability to maximize, to the greatest extent possible, the yield of clinically proven blood components during a single collection procedure.

Minimum yield requirements for certain components are often prescribed by governmental regulations. For example, in the United States, Federal Regulations [Title 21 C.F.R. § 640.24(c)] require the presence, per therapeutic unit of platelets, of at least $5.5 \times 10^{10}$ platelets in at least 75% of the units tested. Typically, a unit of platelets includes, as a suspension media, about 50 milliliters of plasma.

Another desirable feature for a blood collection and separation system and method is the capability of yielding components which are suited for storage for prolonged periods. This feature is closely related to the degree of sterility a given blood collection system can assure. Such matters are also usually the subject of governmental regulations.

For example, in the United States, whole blood and components which are collected and processed in a nonsterile, or "open", system must be transfused within twenty-four (24) hours of collection. On the other hand, in the United States, whole blood and red cells which are collected in a sterile, or "closed", system may be stored for upwards to thirty-five days, depending upon the type of anticoagulant and storage medium used. Likewise, platelets which are collected in a "closed" system may be stored for upwards to five days, and possibly longer, depending upon the ability of the storage container to maintain proper storage conditions. Plasma which is collected in a "closed" system may be frozen for even more prolonged storage periods.

In the United States, Federal Regulations [Title 21 C.F.R. §640.16(b)] define a "closed" blood collection system as one in which the initially sterile blood collection and transfer containers are integrally attached to each other and not open to communication with the atmosphere. Furthermore, to remain a "closed" blood collection system in the United States, the blood collection container of the system cannot be "entered" in a non-sterile fashion after blood collection. An entry into a blood collection system which presents the probability of non-sterility which exceeds one in a million (i.e., greater than $10^{-6}$) is generally considered in the United States to constitute a "non-sterile" entry.

Representative examples of known whole blood collection assemblies include the following United States Patents:

| Earl | 3,064,647 |
| Wandell et al | 3,078,847 |
| Bellamy Jr. | 3,110,308 |
| Tenczar Jr. | 3,187,750 |
| Viguier | 3,870,042 |
| Garber et al | 3,986,506 |
| Djerassi | 4,111,199 |
| Smith | 4,222,379 |

Representative examples of known commercially available whole blood collection assemblies are sold by Fenwal Laboratories, Inc. (a division of Travenol Laboratories, Inc., Deerfield, Illinois); Delmed Corp., Irvine, California; and Cutter Laboratories, Inc., Berkeley, California.

All of the above-identified blood collection assemblies rely exclusively upon nonautomated batch centrifugation procedures to separate the collected unit of whole blood (approximately 450 milliliters) into its various components.

During conventional batch centrifugation, the collected unit of whole blood is first subjected to a centrifugal force for a period of time sufficient to initially separate the whole blood into red blood cells and

**0 116 626**

plasma in which substantial amounts of platelets are present (known as platelet-rich plasma). This step of the procedure is commonly referred to as the "soft spin".

The platelet-rich plasma is then manually expressed into another container and subjected to a greater centrifugal force for generally a longer period of time to further separate the platelet-rich plasma into platelet concentrate and platelet-poor plasma. This step of the procedure is commonly referred to as the "hard spin".

During the course of nonautomated batch centrifugation, approximately 100 milliliters of plasma otherwise suited for storage or further fractionation is "lost", because some of it remains with the red blood cells after the soft spin, and some of it is transferred along with the platelets after the hard spin. Therefore, using conventional batch centrifugation, plasma yields cannot be optimized.

Furthermore, as the authors observe in S. J. Slichter et al. "Preparation and Storage of Platelet Concentrates (Factors Influencing the Harvest of Viable Platelets from Whole Blood)", *British Journal of Haematology,* 1976, 34, 395—402, "accurate standardized centrifugation procedures are critical for efficient preparation of platelet concentrates". To harvest 86% of the platelets from a unit of whole blood without loss of viability, the authors recommend (on page 401 of the article) a soft spin of 1000g (i.e., one thousand times the force of gravity) for 9 minutes, followed by a hard spin of 3000 g for 20 minutes.

Centrifugal procedures which optimize platelet yields thus tend to be time consuming. Furthermore, because skilled technicians are required to calibrate and operate the centrifuges used during these procedures, nonautomated batch centrifugation also tends to be labor intensive.

Additionally, because platelets will lose viability if spun too hard and/or too long, it is extremely difficult, if not impossible, to create centrifugal forces during a conventional hard spin sufficient to separate virtually all of the platelets from the plasma. For example, the Slichter et al article observes (on page 397) that platelet-poor plasma obtained during conventional batch centrifugation techniques includes platelets present in a concentration of between 13,000 and 16,000 platelets per microliter.

The inability of conventional batch centrifugation techniques to yield virtually platelet-free plasma leads to further inefficiencies in blood component processing.

For example, it has been observed that the presence of platelets in the plasma from which Clotting Factor VIII-rich cryoprecipitate is obtained reduces the effective yields of the Factor VIII. Thus, platelet-poor plasma obtained by conventional batch centrifugation techniques does not maximize, to the greatest extent possible, the yield of Factor VIII.

Furthermore, the measurable presence of platelets in platelet-poor plasma means, of course, that these platelets are not present in the platelet concentrate. Thus, conventional batch centrifugation techniques do not maximize, to the greatest extent possible, the yield of platelet concentrate.

Therefore, another desirable feature of blood collection and separation systems and methods is the ability to obtain optimal yields of platelets and virtually platelet-free plasma.

A novel blood collection system which is capable of optimizing component yields using exclusively nonautomated batch centrifugation techniques is disclosed in copending Ronald A. Williams et al, U.S. Patent Application No. 373,555, filed April 30, 1982, the entitled Increased Yield Blood Collection Systems and Methods, which is a continuation-in-part of U.S. Patent Application No. 316,918, filed October 30, 1981.

US—A—4 187 979 discloses a closed blood component collection system comprising: first means for receiving whole from a donor for separation into a first component of substantially red blood cells and a second component of platelet-rich plasma, the first means being suitable for use with an extracorporeal centrifugal blood processing device and including a centrifugation container usable with the processing device to undergo centrifugation and first branch conduit means connected to the centrifugation container for introducing whole blood from a donor into said centrifugation container for separation therein into said substantially red blood cells and platelet-rich plasma in response to centrifugal forces and second means for receiving the platelet-rich plasma from the first means and for separating the plasma into a third component of platelet concentrate and a fourth component of virtually platelet-free plasma.

With the foregoing considerations in mind, one of the principal objects of this invention is to provide blood collection systems and methods which maximize, to the greatest extent possible, the yield of blood components obtained during a single collection procedure in a manner which also assures the maximum available storage period for each of the components collected, as measured by applicable United States standards.

Another principal object of this invention is to provide blood collection systems and methods which, in addition to the above-described attributes, provide plasma which is virtually platelet-free.

Yet another principal object of this invention is to provide blood collection systems and methods which, in addition to any of the above-described attributes, minimize, to the greatest extent possible, the overall time involved during a given procedure.

Yet another one of the principal objects of this invention is to provide blood collection systems and methods which, in addition to the just described attributes, need not depend entirely upon costly, relatively large and sophisticated processing devices.

One aspect of the invention provides a system as disclosed in US—A—4 187 979 as described above, wherein the second means is operative for attachment to pump means and includes microporous membrane means for filtering the cellular components of blood from the non-cellular compounds of blood and second branch means connecting the centrifugation container with said microporous membrane

3

means and attachable to the pump means for introducing the platelet-rich plasma into said microporous membrane means for filtration into platelet concentrate and virtually platelet-free plasma;

and the system comprises a first transfer container, third branch means communicating with said microporous membrane means and said first transfer container for transferring said virtually platelet-free plasma into said first transfer container, a second transfer container and a fourth branch means communicating with said microporous membrane means and said second transfer container for transferring said platelet concentrate into said second transfer container, each of said branch means in said first and second portions of said system comprising a length of flexible tubing integrally connected with said system to form a fluid path which is closed to communication with the atmosphere.

As used herein, the term "platelet-rich plasma" means that platelets are present in a concentration of about twice that normally found in the whole blood of the donor. Generally, the normal concentration of platelets in a healthy adult is about 200,000 platelets per microliter of whole blood. Thus, the associated platelet-rich plasma from this donor would have a platelet concentration of approximately 400,000 platelets per microliter of plasma.

As used herein, plasma is "virtually platelet-free" when the platelet concentration is about 12,000 platelets per microliter of plasma or less. The infusion of platelets in these small concentrations is generally recognized by the medical community to exert no significant therapeutic effect upon the human body, when compared to the therapeutic effect of infusions of platelets in larger concentrations. Thus, the therapeutic value of plasma which is virtually platelet-free is attributable solely to the plasma and plasma-based components other than platelets. Plasma which is virtually platelet-free can itself be infused for therapeutic purposes, or it can be used as source plasma for fractionation purposes.

As used herein, platelets are in "concentrated" form when they are present in a concentration which meets or exceeds the prevailing minimum platelet yield requirements prescribed by governing regulations.

In a preferred embodiment, each of the blood component collecting means includes means for imparting a physical characteristic which is beneficial to the intended function of the associated collecting means. For example, the physical characteristic imparted to the collecting means for the platelet concentrate includes improved gas transmission characteristics for improved platelet survival; the physical characteristic imparted to the collecting means for the virtually platelet-free plasma includes relatively high low-temperature strength to facilitate freezing of the virtually platelet-free plasma; and the physical characteristic imparted to the collecting means for the red blood cells is the suppresion of hemolysis in red blood cells during storage.

The second means includes microporous membrane means operative for filtering the cellular components from the noncellular components of blood. Because the filtration of platelets (a cellular component) from plasma (a noncellular component) can occur virtually instanteously, significant savings in time can be achieved, compared to the time involved in centrifugally separating these components.

The system which embodies the features of the invention is applicable for use in the context of either a nonautomated batch centrifugation process or a continuous flow procedure.

In the continuous flow embodiment, the first means further includes means for returning the red blood cells to the donor. The first means can also include means for diverting a volume of the red blood cells away from the donor for storage. Preferably, the red blood cell diversion means includes the physical characteristic of suppressing hemolysis in red blood cells during storage.

The system comprises a "closed" system, as measured by applicable regulations. Components which are collected in the system can thus be stored for the maximum permissible times.

The invention also provides a method of separating a volume of whole blood received from a donor into components, comprising centrifuging the whole blood to provide a first component of substantially red blood cells and a second component of platelet-rich plasma, and separating the platelet-rich plasma into a third component of platelet concentrate and a fourth component of virtually platelet-free plasma, characterised in that the said platelet-rich plasma is non-centrifugally separated into platelet concentrate and virtually platlet-free plasma by filtering the platelet-rich plasma through a microporous membrane.

The system and method which embody the features of the invention each provide virtually platelet-free plasma, thereby maximizing platelet yields, in a manner which involves significantly less time and manual labour than conventional centrifugation processes.

Other features and advantages of the invention will be pointed out in, or will be apparent from, the specification and claims, as will obvious modifications of the embodiment shown in the drawings.

Fig. 1 is a functional block diagrammatic view of an increased yield blood component collection method which embodies the features of the invention;

Fig. 2 is a functional diagrammatic view of an increased yield blood component collection system which embodies the features of the invention and which can carry out the method shown in Fig. 1;

Fig. 3 is a functional diagrammatic view of another increased yield blood component collection system which embodies the features of the invention and which can carry out the method shown in Fig. 1;

Fig. 4 is an enlarged side view, with portions broken away and in section, of the microporous membrane means which is associated with the systems shown in both Fig. 2 and 3;

Fig. 5 is an end section view, with a portion broken away and in section, of the microporous membrane means taken generally along line 5—5 in Fig. 4;

4

Fig. 6 is a plan view, with portions broken away and in section, of an increased yield blood component collection assembly which embodies the features of the system shown in Fig. 2;

Fig. 7 is a plan view of an increased yield blood component collection assembly which embodies the features of the system shown in Fig. 3;

Fig. 8 is an enlarged view of a portion of the assembly shown in Fig. 6;

Fig. 9 is a further enlarged view, with portions broken away and in section, of a portion of the assembly shown in Fig. 6, showing the connector means associated with the assembly in an uncoupled relationship;

Fig. 10 is an enlarged view, with portions broken away and in section, of the connector means shown in Fig. 9 in a coupled relationship and being exposed to a radiant energy-induced melting apparatus to open a fluid path therethrough; and

Fig. 11 is an enlarged view, with portions broken away and in section, of the connector means shown in Fig. 10 after the fluid path has been opened therethrough.

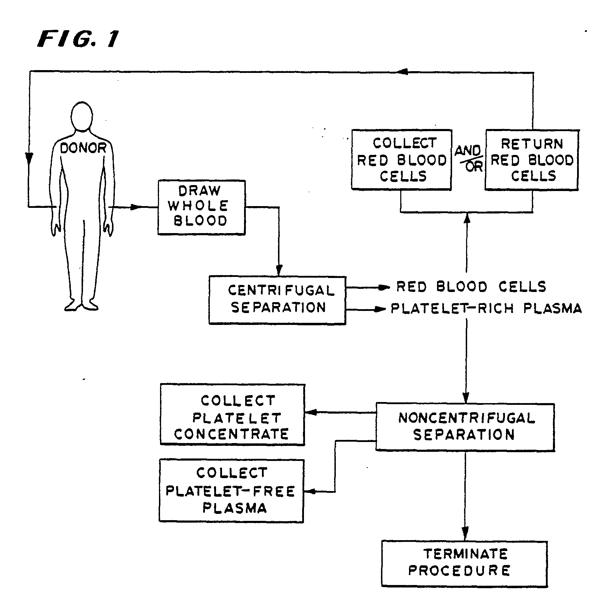

A blood component collection method which embodies the features of the invention is shown in Fig. 1. The method includes the step of separating a volume of whole blood essentially into red blood cells and platelet-rich plasma. The method next includes the step of separating the platelet-rich plasma into platelets and virtually platelet-free plasma.

The method includes the additional steps of collecting the red blood cells, the platelet concentrate and the virtually platelet-free plasma, each for storage or further fractionation.

The method which embodies the features of the invention utilizes the "soft spin" of a conventional nonautomated batch centrifugation technique to initially separate the whole blood into red blood cells and platelet-rich plasma.

The method which embodies the features of the invention also utilizes, in combination with a centrifugal soft spin, the subsequent step of noncentrifugally separating the platelet-rich plasma into the platelet concentrate and virtually platelet-poor plasma by membrane filtration.

The method can also include the step of returning the red blood cells to the donor to achieve a continuous flow arrangement. In this operative environment, the method can optionally include the additional step of diverting a volume of the red blood cells away from the donor for storage purposes.

Each step of the method is done in a manner which does not expose the blood components to communication with the atmosphere. Maximum permissible storage time for each of the components collected is thereby achieved.

The significant features and advantages of the just-described method will become even more apparent after the following detailed description of increased yield blood component collection systems which embody the features of the invention.

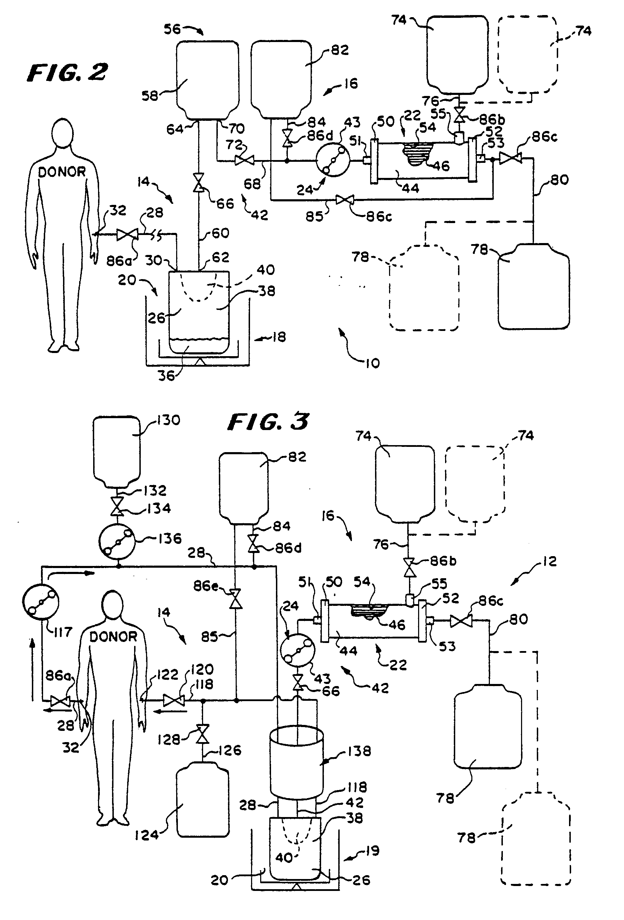

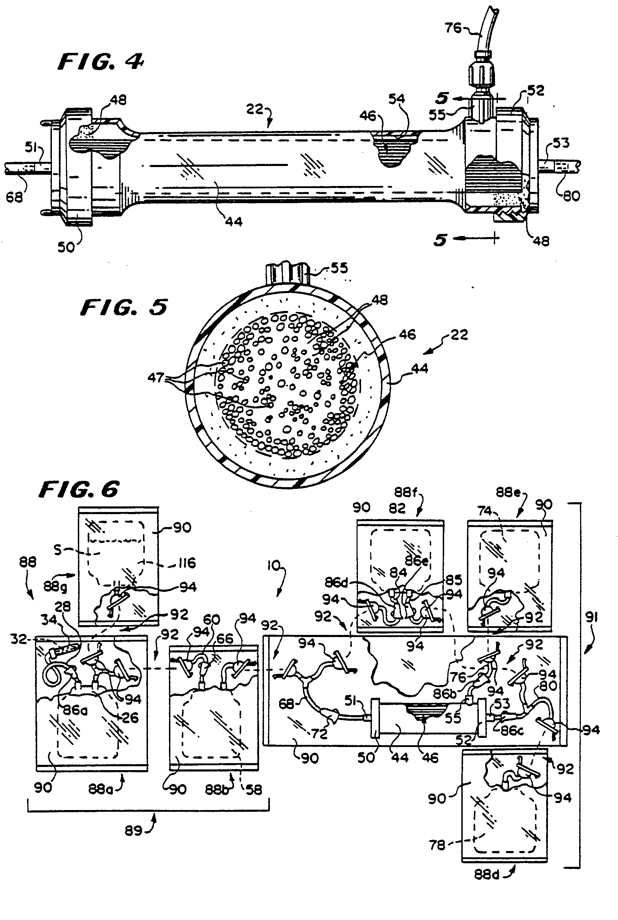

Reference is now made to Figs. 2 and 3. There, blood component collection systems, respectively 10 and 12, are shown. Each of the systems 10 and 12 embodies the features of the invention and can be used to carry out the method shown in Fig. 1. The system 10 shown in Fig. 2 is best suited for use in association with a nonautomated batch centrifugation process. The system 12 shown in Fig. 3 is best suited for use in association with a continuous flow centrifugation process.

Attention is first directed to the system 10 shown in Fig. 2.

As shown in Fig. 2, the system 10 comprises first means 14 or portion for collecting an aliquot, or unit, of whole blood from a donor for separation into essentially red blood cells and platelet-rich plasma. Typically, the aliquot can contain about 450 milliliters of whole blood.

The system 10 further includes second means 16 or portion for receiving the platelet-rich plasma from the first means 14 and for noncentrifugally separating the platelet-rich plasma into platelet concentrate and virtually platelet-free plasma.

The first and second means 14 and 16 may be variously constructed. However, in the illustrated embodiment (which corresponds with the preferred embodiment of the heretofore described method), the first means 14 is operative for use with an extracorporeal batch blood processing device 18 having a centrifugal separation chamber 20, both of which are shown schematically in Fig. 2. The second means 16 employs filtration to effect further separation of the platelet-rich plasma and includes microporous membrane means 22 which is operative for attachment to external pump means 24. More particularly, the microporous membrane means 22 of the second means 16 is operative for filtering the cellular components of blood (such as the platelets) from the non-cellular components (such as plasma).

In this preferred arrangement, the first means 14 includes a centrifugation container 26 which is suited for placement within the separation chamber 20 of the device 18 to undergo centrifugation.

The first means 14 further includes first branch conduit means 28 which communicates, at one end, with an inlet port 30 in the centrifugation container 26. The first branch means 28 communicates, at its other end, with a phlebotomy needle 32. Whole blood is introduced from the donor into the centrifugation container 26 through the first branch means 28.

The phlebotomy needle 32 may be integrally connected with the branch conduit means 28 and be normally sealed from communication with the atmosphere by conventional needle cover 34 or sheath (not shown in Fig. 2, but shown in Fig. 6). The cover 34 is removed at the time of venipuncture.

Alternatively, the branch conduit means 28 can include a conventional needle adaptor (not shown), such as those provided in Fenwal Blood Recipient Sets, sold by Fenwal Laboratories, Inc., a division of Travenol Laboratories, Inc., Deerfield, Illinois. The needle adaptor receives the needle 32 at the time of venipuncture.

To prevent the whole blood introduced into the container 26 from clotting, a volume of a conventional anticoagulant solution 36 (such as ACD or CPD) is carried in the container 26.

The whole blood which is introduced into the container 26 is subjected to a centrifugal force field developed within the chamber 20. Because of known differences in densities, components of the whole blood separate and congregate within the container 20 in different zones radially spaced from the rotational axis of the chamber 20.

The desired separation of the components depends upon the magnitude of the centrifugal forces developed. By using a Sorvall RC-3 centrifuge (HG-4L rotor) at 1900 RPM (1000×g), the whole blood can be separated in approximately 6 minutes into essentially red blood cells (which congregate in the zone 38 of the container 26) and platelet-rich plasma (which congregates in the zone 40 of the container 26).

The second means 16 of the preferred arrangement includes second branch means 42 which delivers the platelet-rich plasma from the zone 40 of the centrifugation container 26 to the microporous membrane means 22. The second branch means 42 includes a portion 43 which is operative for attachment to the pump means 24 for introducing the platelet-rich plasma into microporous membrane means 22.

In this arrangement, the red blood cells are preferably retained in the centrifugation container 26 for storage.

The microporous membrane means 22 may be variously constructed. In the illustrated embodiment, and as best shown in Figs. 4 and 5, the membrane means 22 includes a tubular housing 44 in which a bundle of microporous hollow fiber membranes 46 is mounted. The membranes 46 can be mounted within the housing 44 utilizing conventional potting techniques, such as the one disclosed in Mahon, U.S. Patent 3,228,876.

During the potting operation disclosed in the above patent, a liquid potting compound 48 (for example, polyurethane) is introduced into opposite ends of the housing 44 to impregnate the exterior areas of the membranes 46 about and between the ends 47 of the individual fibers (see Fig. 5). Ingress of the potting compound 48 into the bores of the fiber ends 47 can be prevented by various means, such as those discussed in the above-cited Mahon patent.

After the potting compound 48 has cured and the fiber ends 47 opened, end caps 50 and 52 may be sealed to the potted ends of the housing 44. An inlet port 51 is formed on the end cap 50, and an outlet port 53 is formed on the end cap 52.

Circumferentially surrounding the bundle of hollow fiber membranes 46 is an open volume 54 (see Fig. 4) which is sealed at each end by the cured potting compound 48. An outlet port 55 communicates with the volume.

Materials from which the microporous fiber membranes 46 can be made to accomplish the separation of cellular components of blood from noncellular components include certain thermoplastic polymers such as polypropylene. These materials can be formed into hollow fibers by known processes such as solution spinning or melt spinning.

For example, a polypropylene hollow fiber can be manufactured which has a wall thickness of approximately 150 microns, an interior diameter of approximately 320 microns, a maximum pore size of approximately .55 microns, and an average pore size of approximately .30 microns. Such a hollow fiber is commercially available from Enka A. G., the Federal Republic of Germany, and is well-suited for the purposes herein described.

In an alternate embodiment (not shown), the membrane means 22 can take the form of a device having spaced-apart, generally planar membranes made of the same or comparable microporous material. An example of such a device is disclosed in Edelman et al, U.S. Patent 4,313,813.

The second branch means 42 can be operative for transferring the platelet-rich plasma directly from the centrifugation container 26 to the microporous membrane means 22 (see, e.g., the system 12 in Fig. 3). However, in the Fig. 2 embodiment, the second branch means 42 includes means 56 for temporarily collecting or pooling the platelet-rich plasma prior to filtration. The pooling means 56 includes a pooling container 58 and first transfer conduit means 60 which communicates, at one end, with an outlet port 62 which communicates with the zone in the centrifugation container 26 and, at the other end, with an inlet port 64 in the pooling container 58.

The pooling means 56 further includes valve means 66, such as a manual roller clamp or hemostat, inline with the first transfer conduit means 60 to control the transfer of the platelet-rich plasma. This transfer can be accomplished, after centrifugation, by manually expressing the platelet-rich plasma from the container 26. A pump (not shown) may be used for the same purpose.

The pooling means 56 also includes, in this arrangement, second transfer conduit means 68 which communicates, at one end, with the inlet port 51 of the membrane means 22 and, at the other end, with an outlet port 70 in the pooling container 58. By operatively connecting the portion 43, which in the illustrated embodiment is situated along the second transfer conduit means 68, with the external pump means 24, the platelet-rich plasma can be introduced into the membrane means 22 for filtration.

Valve means 72, again such as a roller clamp or hemostat, is provided inline with the second transfer conduit means 68 for controlling this transfer.

The pump means 24 may be variously constructed. However, in order to meet all of the collection objectives of the system 10, operative contact between the conduit portion 43 and the pump means 24 must not compromise the sterile integrity of, or otherwise "open", the system, as judged by appliable standards in the United States.

In the illustrated embodiment, the pump means 24 takes the form of a conventional peristaltic pump, such as one manufactured and sold by Renal Systems under the trade name Minipump. The pump serves to repeatedly compress and expand the tubing portion and cause whole blood to flow into the hollow fiber membranes 46.

By controlling the pump speed, the proper flow characteristics within the hollow fiber membranes 46 can be maintained to cause effective separation of the cellular components (i.e., the platelets) from the noncellular component (i.e., the plasma).

Plasma which is virtually free of platelets collects in the open volume 54. Meanwhile, platelets, in concentrated form, exit the outlet port 53.

Because the separation of virtually all of the platelets from the platelet-rich plasma occurs essentially simultaneously as the platelet-rich plasma traverses the membrane means 22, significant economies of time can be realized using the system 10. Furthermore, because the system 10 requires only a peristaltic pump or the like to perform the second separation step, economies of labor can also be achieved.

Just as significantly, because the plasma which collects in the volume 54 is virtually platelet-free, the number of platelets present in the concentrate which exits the port 53 is maximized by the system 10 to the greatest extent possible.

The second means 16 also includes a first transfer container 74 or containers (shown in phantom lines) and associated third branch means 76 which transfers the virtually platelet-free plasma from the volume 54 into the first transfer container 74 for storage, or for further fractionation into Factor VIII-rich cryoprecipitate.

The second means 16 also includes a second transfer container 78 or containers (shown in phantom lines) and associated fourth branch means 80 for transferring the platelet concentrate from the outlet port 53 into the second transfer container 78 for storage.

The system 10 also preferably includes a source 82 of sterile saline solution and branch conduit means 84 which communicates with the second means 16 upstream of the microporous membrane means 22. The saline is used to prime the membrane means 22 and purge air from the second means 16 prior to collection. Vent conduit means 85 is preferably provided to circulate the priming volume of saline back to the saline source 82.

Additional valve means 86a, b, c, d, and e are provided inline with, respectively, the first, third and fourth branch means 28, 76, and 80, as well as the priming branch conduit means and vent conduit means 84 and 85, to direct the flow of fluids through the system 10 to carry out the method shown in Fig. 1. The valve means 86a through e may take the form of manually actuated roller clamps or hemostats.

To further enhance the storage of the components which are collected by the system 16, the centrifugation container 26 (in which the red blood cells are collected), the first transfer container 74 (in which the virtually platelet-free plasma is collected), and the second transfer container 78 (in which the platelet concentrate is collected) are each purposely imparted with a predetermined physical characteristic which is beneficial to the intended storage function of the container. This aspect of the invention will be described in greater detail later herein.

In the embodiment shown in Fig. 2, the first, second, third, and fourth branch conduit means 28, 42, 76, and 80, as well as the priming branch conduit means 85 and vent conduit means 84 and 85, each takes the form of a length of flexible tubing or conduit made of a plastic hemocompatible material, such as plasticized polyvinyl chloride.

Each length of tubing or conduit offers a fluid path which is closed from communication with the atmosphere, and the system 10 is not thereafter "entered" in a non-sterile fashion as the blood collection procedure takes place. The system 10, once sterilized, thus constitutes a sterile, "closed" system, as judged by applicable standards in the United States. The collected components can thus be stored for the maximum permissible time.

The system 10 shown in Fig. 2 can be manufactured and sold in various configurations. An illustrative assembly 88 is shown in Fig. 6.

In Fig. 6, the assembly 88 is initially configured into seven separate subassemblies (designated 88a through g). The subassemblies 88a through g can be coupled together by the operator to form the system 10 shown in Fig. 2.

In this arrangement, each subassembly 88a through g is preferably housed in a tear-away protective overwrap 90 in which each subassembly is sterilized. The overwraps 90 are removed at time of use. The overwrap 90 also preferably prevents evaporation of fluids present in any of the subassemblies prior to use (such as saline in the subassembly 88f).

In this arrangement, the assembly 88 further includes means 92 for selectively establishing fluid paths among the normally separate subassemblies 88a through g in a manner which does not compromise the sterile closed integrity of the subassemblies or of the formed system 10 as a whole.

More particularly, the means 92 includes one or more connector means 94 associated with each subassembly. The connector means 94 is carried within the confines of the tear-away overwrap 90 prior to use.

# 0 116 626

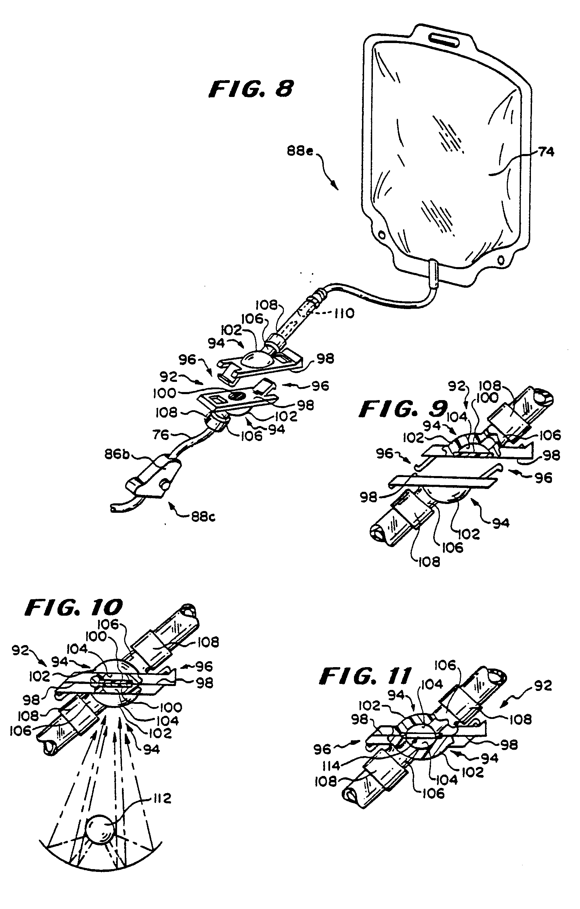

As can best be seen in Figs. 8 through 11, each connector means 94 includes means 96 for selectively mechanically coupling pairs of the connector means together with a portion 98 of each in facing contact. The facing portions 98 include means 100 operative for melting to form a fluid path through the joined pairs of the connector means 94, thereby opening fluid communication between the various subassemblies, but only in response to exposure to an energy source efficient in itself to effectively sterilize the means 100 as they melt. This constitutes an active sterilization step which occurs simultaneously with the formation of the fluid path.

Furthermore, during the act of melting, the means 100 are preferably operative for fusing together to form a hermetic seal about the periphery of the fluid path. The resulting connection is thus internally sterile and closed from communication with the atmosphere.

The connector means 94 may be variously constructed and employ different means of operation. However, to meet the desired increased-yield objectives of the system 10, the connector means 94 each must meet certain operative requirements.

More particularly, each connector means 94 must (1) normally close the associated subassembly from communictaion with the atmosphere; (2) be opened only in conjunction with an active sterilization step which serves to sterilize the regions adjacent to the fluid path as the fluid path is formed; and (3) be capable hermetically sealing the fluid path at the time it is formed.

It has been determined that the sterile connector generally described in Granzow et al U.S. Patents 4,157,723 and 4,265,280 meets all of the above criteria and, for this reason, such a connector is shown in the illustrated embodiment.

The construction and operation of such a connector can be best seen in Figs. 9 through 11, where the connector means 94 used to join the subassembly 88e with the subassembly 88c are shown. It should be appreciated that all of the connector means 94 associated with the other subassemblies operate in the identical fashion.

More particularly, each connector means 94 includes a housing 102 which defines a hollow interior 104 which communicates with its associated subassembly. The heretofore described meltable means 100 takes the form of a meltable wall which normally seals or closes the associated interior, and thus subassembly, from communication with the atmosphere.

The housing further includes a tubular conduit portion 106 which communicates with the inteior 104 and which serves to interconnect the connector means 94 to the length of tubing which forms a part of the associated subassembly.

While the connector means 94 may be variously attached to the end of the tubing, in the illustrated embodiment, a hermetic, friction fit between the tubular conduit portion 106 is envisioned. An elastic band 108, such as made from a latex material, preferably encircles the outer periphery of the junction to assure a fluid tight, hermetic fit between the tubular portion 106 and the respective tubing.

To normally prevent fluid flow communication with the interior of the connector means 92 in this arrangement, an inline valve member 110 (shown in phantom lines in Fig. 8) may be provided. Such an arrangement is particularly desirable in association with any fluid-filled container.

While the valve member 110 may be variously constructed, in the illustrated embodiment, it takes the form of an inline frangible valve member, such as one disclosed in Bayham et al, U.S. Patents No. 4,181,140 and 4,294,247.

Alternately, the frangible valve member 110 can form an integral part of the connector housing 102, as is shown in Granzow et al, U.S. Patent 4,265,280.

In the illustrated embodiment, the meltable wall 100 is fabricated from a radiant energy absorbing material. It is thus operative for melting in response to exposure to a source of radiant energy. Furthermore, the material from which the wall 100 is constructed may be purposefully preselected so that it melts only at temperatures which result in the rapid destruction of any bacterial contaminant on the surface of the material (i.e., over 200°C). To permit the transmission of radiant energy through the housing 102 to the meltable wall 100, the housing 102 is made of a material which minimizes absorption of the particular type of radiant energy selected.

In the preferred embodiment, the wall 100 is made of a material fabricated from poly(4 - methyl - 1 - pentene), which is sold under the trademark TPX by Mitsui Chemical Company. This material has a crystalline melting point of approximately 235°C, and is further discussed in Boggs et al U.S. Patent 4,325,417. The material of the wall 100 is colored black so as to absorb infrared radiation. The housing 102 is made of a clear TPX material which is generally transparent to the passage of radiation.

As can be best seen in Fig. 8, the connecting means 96 takes the form of mating bayonet-type coupling mechanisms, which serve to interlock a pair of connector means 94 together with their radiant energy absorbing walls 100 in facing contact (see Fig. 10). When exposed to a radiant energy source which, in the illustrated embodiment, consists of an incandescent quartz lamp 112 focused on the opaque wall 100, the radiant energy absorbing walls 100 melt and fuse together, as can be seen in Fig. 11. In the process of melting, the walls 100 form a hermetically sealed opening 114 which establishes through the connector means 94 a fluid path which is at once sterile and closed to communication with the atmosphere.

As the following Example 1 demonstrates, the utilization of the illustrated connector means 94 assures a probability of non-sterility which exceeds $10^{-6}$.

8

Example 1

A methanol suspension of $1.5\times10^8$ *Bacillus subtilis* var *niger* (globiguii) spores per milliliter was prepared. This organism was chosen because of its high resistance to dry heat (see Angelotti, et al, "Influence of Spore Masture Content on the Dry Heat Resistance of *Bacillus subtilis* var *niger"*, Appl. Microbiol., v 16 (5): 735—745, 1968).

Eighty (80) uncoupled sterilized connector members (i.e., forty (40) pairs) identical to the connector means 94 shown in Figs. 8 through 11, were inoculated with 0.01 milliliter of the *B subtilis* var *niger* (globiguii) suspension. This constituted exposure of the associated walls 100 of each connector member to approximately one million (i.e., $10^6$) spores of the organisms.

Forty (40) of the inoculated uncoupled connectors were each attached to empty, sterile containers. The other forty (40) were each attached to containers containing a sterile microbiological growth medium (soybean casien digest (SCD) broth). These inoculated pairs of connector members will hereafter be referred to as the Test Connectors.

Sixteen (16) additional uncoupled and sterilized connector members (i.e., eight (8) pairs) were inoculated only with methanol. Eight (8) of the connectors were each attached to empty, sterile containers, and eight (8) were each attached to sterile containers containing the SCD broth. These will hereafter be referred to as Negative Control Connectors.

The Test Connectors were coupled together, forming forty (40) connections between the empty containers in the SCD broth containers. The noninoculated Negative Control Connectors were also coupled together, forming eight (8) connections between the empty containers and the SCD broth containers. Each connection was placed within the light-induced melting apparatus as heretofore described to fuse the membranes together and open a fluid path. The medium was then passed through the connections.

Eight (8) additional and already fused connector members were inoculated as Positive Controls. Two of these connections were inoculated with a theoretical challenge of $10^6$ *B subtilis* var *niger* (globiguii) spores per connection; two were inoculated with a theoretical challenge of $10^4$ spores per connection; two were inoculated with a theoretical challenge of $10^2$ spores per connection; and two were inoculated with a theoretical challenge of $10^1$ spores per connection. Medium was then flushed through the fluid path of these Positive Control Connectors.

All units were incubated at approximately 32° to 37°C for up to seven days. After incubation all turbid broths were subcultured to SCD agar and incubated for 18 to 24 hours at approximately 32° to 37°C. The subcultures were examined for the presence of orange colonies, which is characteristic of the indicator organism.

Upon examination of the forty (40) Test Connections, no turbid broths were observed.

All eight (8) Negative Controls also remained negative during incubation.

All eight (8) Positive Controls demonstrated growth of the indicator organism at all inoculum levels.

Referring again to the assembly 88 shown in Fig. 6, it should be appreciated that two or more of the various subassemblies 88a through g could be integrally joined together during manufacture, thereby reducing the overall number of subassemblies to be joined by the operator.

For example, in an alternate arrangement, the subassemblies 88a and 88b (comprising the centrifugation container 26 and pooling container 58) could constitute an integrally joined unit 89. The unit 89 could be placed in the centrifugation chamber 20.

In this alternate arrangement, the subassemblies 88c, d, and e (comprising the first and second transfer containers 74 and 78 and the microporous membrane means 22) could also be another integrally joined unit 91 separate from the unit 89. The unit 91 would remain outside of the centrifugal chamber during centrifugation, and would be joined to the unit 89 (using a pair of the connector means 94) after centrifugation has taken place.

After the various components have been collected in the containers 26, 74 and 78, each container is preferably sealed closed and separated from the system 10. This can be accomplished utilizing conventional means, such as a spaced-apart pair of hand seal clips (not shown), or by the formation of a hermetic, snap-apart seal using a Hematron® dielectric sealer (also not shown) sold by Fenwal Laboratories.

As before explained, in a preferred embodiment, at least a portion of each of the component collection containers 26, 74, and 78 is purposely imparted with a predetermined physical characteristic which is beneficial to the intended storage function of the particular container.

For example, to maximize the storage times of the virtually platelet-free plasma, the transfer container 74 is preferably made of a material having a relatively high low-temperature strength to withstand freezing of the platelet-free plasma for prolonged storage.

Candidate materials for this purpose includes various polyolefin materials, such as low density polyethylene and copolymers of polyethylene and polypropylene, including those containing a major amount of polypropylene.

To maximize the allowable storage time of the platelet concentrate collected in the system 10, the transfer container 78 in which platelet concentrate will be stored preferably has a gas transfer characteristic beneficial to prolonged platelet storage. More particularly, the transfer container 78 would preferably have a gas transfer characteristic which exceeds that of polyvinyl chloride plasticized with di - 2 - ethylhexylphthalate (DEHP).

9

**0 116 626**

For example, the transfer container 78 can include a polyolefin-type container which is disclosed in Gajewski et al, U.S. Patent 4,140,162, or a polyvinyl chloride container which has been plasticized with tri - 2 - ethylhexyl trimellitate (TEHTM), as disclosed in Warner et al. U.S. Patent 4,280,497.

Alternately, or in addition, the platelet transfer container 78 can include a platelet storage media (not shown) which is suited for maintaining platelet viability during storage.

To enhance the storage of the red blood cells, the centrifugation container 26 is preferably made of a material which is known to suppress hemolysis in red cells during storage. Candidate materials for this purpose include polyvinyl chloride plasticized with di - 2 - ethylhexylphthalate (DEHP).

Alternately, or in addition, an additional transfer container 116 (shown in Fig. 6 as a part of the subassembly 88g) can be attached to the centrifugation container 26 utilizing the connector means 94. This transfer container 116 includes an isotonic red cell storage solution (designated "S" in Fig. 6) which is suited for suppressing hemolysis during storage. This solution S would be introduced into the red blood cells remaining in the container 26 after the platelet-rich plasma has been removed.

The solution S could include ingredients such as saline, adenine, mannitol, and glucose, such as the solution disclosed in Grode et al, U.S. Patent 4,267,269, or in copending Grode et al, U.S. Patent Application No. 377,110 filed May 11, 1982, and entitled Red Cell Storage Solution and Method.

Because red cells are collected utilizing the system 10 (and associated assembly 88), the system 10 can be used to collect and process whole blood from an individual donor once every eight weeks in the United States.

Attention is now directed to the blood component collection system 12 shown in Fig. 3. The system 12 shares many common elements with the system 10 shown in Fig. 2. Common reference numerals are provided for these common elements.

Like the system 10 in Fig. 2, the system 12 includes the first means 14, or portion, for collecting whole blood for separation into essentially red blood cells and platelet-rich plasma. In the system 12, however, pump means 117 is utilized to deliver blood into the centrifugation container 26.

Also like the system 10, the system 12 includes the second means 16, or portion, for receiving the platelet-rich plasma from the first means and for noncentrifugally separating the platelet-rich plasma into platelet concentrate and virtually platelet-free plasma.

Like the system 10, the second means 16 of the system 12 includes the microporous membrane means 22 for filtering the cellular component of blood from the noncellular components.

However, unlike the system 10 shown in Fig. 2, the first means 14 of the system 12 is operative for use with an extracoporeal blood processing device 19 which is capable of processing blood in a continuous flow procedure. An example of such a device is found in Cullis et al, U.S. Patents 4,146,172 and 4,185,629.

Examples of commercially available devices and the CS-3000® Blood Cell Separator and the Celltrifuge II® Blood Cell Separator, both of which are manufactured and sold by Fenwal Laboratories.

To accommodate the continuous flow procedure, the first means 14 of the system 12 includes fifth branch means 118 which communicates with the centrifugation container 26 for returning the red blood cells to the donor. Valve means 120 is provided inline with the fifth branch means 118 to control the return.

The fifth branch means 118, like all of the heretofore described branch means, preferably takes the form of a length of flexible tubing or conduit made from a hemocompatible material. The fifth branch means 118 includes, at its terminus, another phlebotomy needle 122.

While individual phlebotomy needles 32 and 122 are shown in Fig. 3, it should be appreciated that the first and fifth branch means 28 and 118 could each communicate in common with one double lumen needle of conventional construction (not shown).

The system 12 shown in Fig. 3 optionally includes a third transfer container 124 and sixth branch means 126 for diverting a volume of the red blood cells traversing the fifth branch means 118 away from the donor and into the third transfer container 124 for storage. Valve means 128 is provided inline with the sixth branch means 126 for controlling the transfer of red blood cells into the container 124.

The system 12 shown in Fig. 3 also includes, in addition to the source 82 of sterile saline and associated branch conduit means 84 and 85, a source 130 of sterile anticoagulant solution and branch conduit means 132 for introducing the anticoagulant solution into the system. Valve means 134 is provided inline with the branch conduit means 132 for controlling the introduction of anticoagulant.

Pump means 136, preferably in the form of a peristaltic pump, can be included to meter the introduction of anticoagulant through the branch means 132.

As in the Fig. 2 system 10, the system 12 is preferably closed to communication with the atmosphere throughout the procedure, thereby maximizing the allowable storage times.

To maintain the closed integrity of the system 12 during the continuous flow procedure, the first, second, and fifth branch conduit means 28, 42, and 118, which all communicate with the centrifugation container 26, are consolidated in an umbilicus 138. According to the principles discussed in Adams, U.S. Patent RE 29,738, this umbilicus 138 is preferably rotated during centrifugation at a speed one-half the speed of the container 26. Twisting of the umbilicus 138 is thereby avoided, and fluid communication through the umbilicus 138 is continuously maintained without the use of rotating seals.

It should be appreciated that the system 12 can be readily adapted for use with a device employing a rotating seal arrangement or the like. However, such an arrangement might compromise the closed sterile

10

integrity of the system 12 as measured by applicable standards in the United States and therefore lead to significantly shortened storage times.

Also unlike the system 10 shown in Fig. 2, the second branch means 42 of the system 12 does not utilize the pooling means 56, but rather transfers the platelet-rich plasma continuously from the container 26 to the microporous membrane means 22 in response to operating the pump means 24.

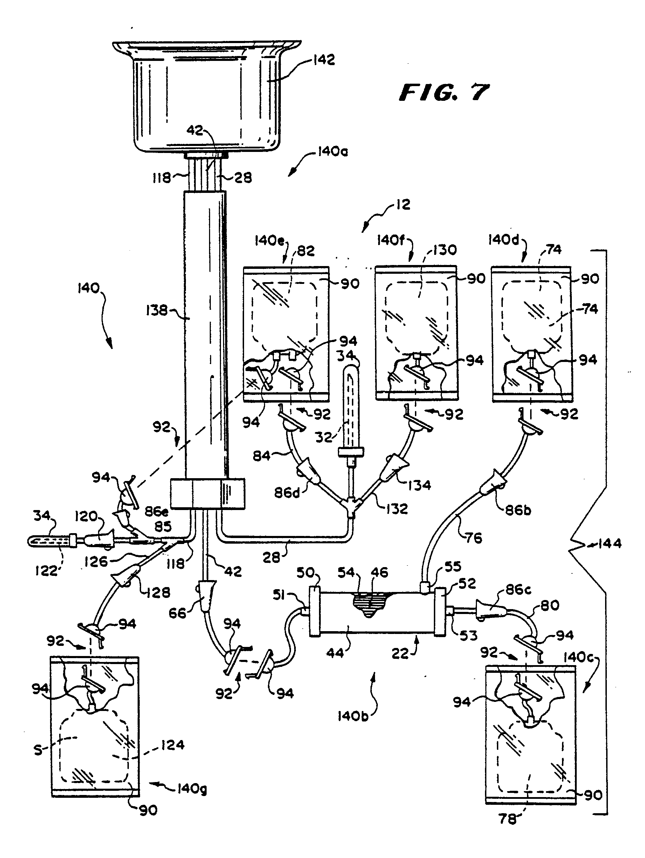

The system 12 shown in Fig. 3 can be variously constructed. In Fig. 7, a representative assembly 140 of the system 12 is shown.

Similar to the assembly 88 shown in Fig. 6, the assembly 140 includes two or more separate subassmblies (designated 140a through g) which can be selectively joined together by the operator to form the system 12 utilizing the heretofore described connector means 94.

All or some of the subassemblies 140a through g can be carried in the protective overwrap 90. Other elements common to the system 10 and corresponding assembly 88, as well as to the system 12, are assigned common reference numerals in Fig. 7.

In the Fig. 7 assembly, the centrifugation container 26 takes the form of a disposable bowl 142 which fits in the centrifugation chamber of the Celltrifuge II® Blood Cell Separator. Specific details of the construction and operation of the bowl 142 are disclosed in copending Bachehowski et al. U.S. Patent Application No. 243,981, filed March 16, 1981 and entitled Centrifugal Processing Apparatus and Rotatable Processing Bowl Apparatus; and Bacehowski et al, U.S. Patent Application No. 244,398, filed March 16, 1981 and entitled Rotatable Bowl Assembly for Centrifugal Processing Apparatus Having a Bonded and Prewound Umbilical System. The disclosure of both of these applications is incorporated herein by reference.

As before explained in the context of the asembly 88, two or more of the subassemblies 140a through g could be integrally joined during manufacture to reduce the overall number of subassemblies.

For example, the subassemblies 140b, c, and d (constituting the membrane means 22 and transfer containers 74 and 78) could constitute an integrally joined unit 144.

One or more of the remaining subassemblies 140e, f, and g (constituting the saline and anticoagulant sources 82 and 130 and transfer container 124) could also be integrally joined to the subassembly 140a (constituting the disposable bowl 142 and associated tubing).

Use of the system 12 and associated assembly 140 permits, during a single continuous flow procedure, the collection of the maximum allowable amounts of virtually all of the therapeutic plasma-based components, and (optionally) a unit of red blood cells. Because the system 12 and assembly 140 each comprise a closed system as judged by United States Standards, all of the collected components are suited for storage for the maximum allowable period.

As before explained, storage of the components can be further enhanced by preselecting the physical characteristics of the containers 74, 78, and 124 to benefit the intended storage function. The red cell storage solution S can also be carried within the third transfer container 124 for intermixing with the collected red blood cells.

If the operator does not collect the optional unit of red cells using the system 12 or assembly 140, the collection procedure can be repeated generally twice a week. If the red blood cells are collected, the procedure can be repeated generally once every eight weeks.

The total volume of components which can be collected in either of the systems 10 or 12 (and each corresponding assemblies 88 and 140) during a given procedure will depend upon the physiology of the donor and maximum allowable total volume permitted by governing regulations.

Nevertheless, either of the systems 10 or 12 is capable of providing optimal yields of platelet concentrate and virtually platelet-free plasma with significant savings in time and labor, when compared to conventional blood collection systems.

Example 2

A unit of whole blood (approximately 450 milliliters) was collected from a healthy donor. The unit was subjected to a "soft spin" of $1000 \times g$ for about 6 minutes.

Whole blood was thereby separated into approximately 200 milliliters of red blood cells and approximately 250 milliliters of platelet-rich plasma.

The 250 milliliters of the platelet-rich plasma was next passed through a device such as shown in Figs. 4 and 5 having approximately 800 polypropylene microporous hollow fibers, each fiber having an effective length of approximately 214 millimeters and a maximum pore size of approximately .55 microns. An inlet flow rate of 50 milliliters per minute at an inlet pressure of less than 50 millimeters Hg was maintained for five minutes.

The platelet-rich plasma was thereby separated into approximately 50 milliliters of platelet concentrate containing approximately $6.5 \times 10^{10}$ platelets (which represents a platelet concentration of approximately $1.3 \times 10^6$ platelets per microliter) and 200 milliliters of plasma having a platelet concentration of only about 120 platelets per microliter (thereby constituting virtually platelet-free plasma).

The following Table summarizes the yields and time of the procedure described in Example 2 and compares these to representative yields obtained by conventional nonautomated batch centrifugal processing.

**0 116 626**

TABLE

| | Platelet concentration in plasma | Platelet concentration in platelet concentrate | Overall elapsed time |
|---|---|---|---|
| Conventional[1] | Approximately 15,000 per microliter (Footnote 2) | Approximately $1.4 \times 10^6$ per microliter (Footnote 2) | About 29 minutes (Footnote 3) |
| Example 2 | Approximately 120 per microliter | Approximately $1.3 \times 10^6$ per microliter | 11 minutes |

[1] Based upon Slichter et al, "Preparation and Storage of Platelet Concentrate (Factors Influencing the Harvest of Viable Platelets from Whole Blood)", British Journal of Haematology, 1976, 34, 395—402.

[2] Slichter et al, *Ibid*, p. 397

[3] Assuming Slichter et al's preferred sequence of a soft spin of 1000 g for 9 minutes and a hard spin of 3000 g for 20 minutes.

**Claims**

1. A closed blood component collection system comprising first means (14) for receiving whole blood from a donor for separation into a first component of substantially red blood cells and a second component of platelet-rich plasma, the first means (14) being suitable for use with an extracorporeal centrifugal blood processing device (18) and including a centrifugation container (26) usable with the processing device to undergo centrifugation and first branch conduit means (28) connected to the centrifugation container for introducing whole blood from a donor into said centrifugation container for separation therein into substantially red blood cells and platelet-rich plasma in response to centrifugal forces; and second means (16) for receiving the platelet-rich plasma from the first means and for separating the plasma into a third component of platelet concentrate and a fourth component of virtually platelet-free plasma; characterised in that: the second means (16) is operative for attachment to pump means (24) and includes microporous membrane means (22) for filtering the cellular components of blood from the non-cellular components of blood and second branch means (42) connecting the centrifugation container with said microporous membrane means and attachable to the pump means for introducing the platelet-rich plasma into said microporous membrane means for filtration into platelet concentrate and virtually platelet-free plasma; and the system comprises a first transfer container (74), third branch means (76) communicating with said microporous membrane means and said first transfer container for transferring said virtually platelet-free plasma into said first transfer container, a second transfer container (78), and fourth branch means (80) communicating with said microporous membrane means and said second transfer container for transferring said platelet concentrate into said second transfer container; and each of said branch means in said system comprises a length of flexible tubing integrally connected with said system to form a fluid path which is closed and does not communicate with the atmosphere.

2. A blood component collection system according to Claim 1, wherein each of said centrifugation containers and first and second transfer containers (26, 78, 74) includes means for imparting a physical characteristic which is beneficial to the intended storage function of the collection system, such as improved gas transmission characteristics for improved platelet survival, relatively high low-temperature strength to facilitate freezing of the virtually platelet-free plasma, and suppression of hemolysis in red blood cells.

3. A blood component collection system according to Claim 1 or 2 wherein said first means (14) includes fifth branch means (118) integrally connected with said system and communicating with the centrifugation container and being operative for returning the red blood cells to the donor.

4. A blood component collection system according to Claim 3, wherein said first means (14) includes a third transfer container (124) and sixth branch means (126) communicating with the return means (118) and the third transfer container for diverting a volume of the red blood cells away from the donor for transfer into the third transfer container.

5. A blood component collection system according to any one of the preceding claims and further including a source of sterile anticoagulant solution (36) and a source (82) of sterile saline solution, means integrally connected to said system for introducing the anticoagulant solution into said system, and further branch means (84) integrally connected to said system for introducing the saline solution into said system.

6. A method of separating a volume of whole blood received from a donor into components, comprising centrifuging the whole blood to provide a first component of substantially red blood cells and a second component of platelet-rich plasma, and separating the platelet-rich plasma into a third component of platelet concentrate and a fourth component of virtually platelet-free plasma; characterised in that the said platelet-rich plasma is non-centrifugally separated into platelet concentrate and virtually platelet-free plasma by filtering the platelet-rich plasma through a microporous membrane.

12

**Patentansprüche**

1. Geschlossenes Blut-Komponenten-Sammelsystem umfassend eine erste Anordnung (14), um Vollblut von einem Spender zur Trennung in eine erste Komponente aus im wesentlichen roten Blutkörperchen und eine zweite Komponente aus an Blutplättchen-reichem Plasma zu erhalten, wobei die erste Anordnung (14) für die Verwendung einer extrakorporalen Blutzentrifugiereinrichtung (18) geeignet ist und einen Zentrifugierbehälter (26), der mit der Zentrifugiereinrichtung zur Durchführung des Zentrifugierens verwendbar ist, sowie eine erste Zweigleitung (28) aufweist, die mit dem Zentrifugier-behälter verbunden ist, um das Vollblut von einem Spender zur Trennung in im wesentlichen rote Blutkörperchen und in Blutplättchen-reiches Plasma durch Zentrifugalkräfte zu leiten; und eine zweite Anordnung (16), um das Blutplättchen-reiche Plasma aus der ersten Anordnung zu erhalten und um das Plasma in eine dritte Komponente, Blutplättchen-Konzentrat, und in eine vierte Komponente, praktisch Blutplättchen-freies Plasma, aufzutrennen dadurch gekennzeichnet, daß die zweite Anordnung an eine Pumpe (24) anschließbar ist und eine Mikroporen-Membran (22) zum Filtrieren der zellulären Blutbestandteile von den nicht-zellulären Blutbestandteilen sowie eine zweite Zweigleitung (42) aufweist, die den Zentrifugierbehälter mit der Mikroporen-Membran verbindet und an die Pumpe anschließbar ist, um das Blutplättchen-reiche Plasma zur Filtration in Blutplättchen-Konzentrat und praktisch Blutplättchen-freies Plasma in die Mikroporen-Membran einzuleiten, und dadurch, daß das System folgende Elemente aufweist:

einen ersten Übertragungs-Behälter (74),

eine dritte Zweigleitung (76), die die Mikroporen-Membran und den ersten Übertragungsbehälter verbindet, um das praktisch Blutplättchen-freie Plasma in den ersten Übertragungs-Behälter zu transferieren,

einen zweiten Übertragungsbehälter (78) und

eine vierte Zweigleitung (80), die die Mikroporen-Membran mit dem zweiten Übertragungsbehälter verbindet, um das Blutplättchen-Konzentrat in den zweiten Übertragungsbehälter zu transferieren;

wobei jede der Zweigleitungen im System ein Stück flexiblen Schlauchs umfaßt, der fest mit dem System verbunden ist, um eine Strömungsbahn zu bilden, welche geschlossen ist und nicht mit der Atmosphäre in Verbindung steht.

2. Blut-Kompontenten-Sammelsystem nach Anspruch 1, dadurch gekennzeichnet, daß die Zentrifugier-Behälter wie auch der erste und zweite Übertragungsbehälter (26, 78, 74) Mittel aufweisen, die eine für die geplante Lagerfunktion des Sammelsystems vorteilhafte Eigenschaft verleihen, wie verbesserte Gas-Transmissions-Eigenschaften für verbessertes Blutplättchen-Überleben, relativ hohe Niedrigtemperatur - Festigkeit, um das Tiefgefrieren des praktisch Blutplättchenfreien Plasmas zu erleichtern, und Unterdrückung der Hämolyse in roten Blutkörperchen.

3. Blut-Komponenten-Sammelsystem nach Anspruch 1 oder 2, dadurch gekennzeichnet, daß die erste Anordnung (14) eine fünfte Zweigleitung (118) aufweist, die fest mit dem System verbunden ist, mit dem Zentrifugier-Behälter in Verbindung steht und der Rückführung der roten Blutkörperchen zu dem Spender dient.

4. Blut-Komponenten-Sammelsystem nach Anspruch 3, dadurch gekennzeichnet, daß die erste Anordnung (14) einen dritten Übertragungsbehälter (124) und eine sechste Zweigleitung (126) aufweist, die die rückführende Leitung (118) und den dritten Übertragungsbehälter miteinander verbindet, um ein Volumen roter Blutkörperchen vom Spender abzuleiten und in den dritten Übertragungsbehälter zu transferieren.

5. Blut-Komponenten-Sammelsystem nach einem der vorangehenden Ansprüche, dadurch gekennzeichnet, daß es darüberhinaus eine Quelle für eine sterile Antikoagulans-Lösung (36), eine Quelle (82) für eine sterile Salzlösung, fest mit dem System verbundene Mittel um die Antikoagulans-Lösung in das System einzubringen sowie eine fest mit dem System verbundene Zweigleitung (34) zur Einleitung der Salzlösung in das System umfaßt.

6. Verfahren zur Auftrennung von Vollblut, das von einem Spender erhalten wurde, in Komponenten, umfassend das Zentrifugieren des Vollblutes, um eine erste Komponente aus im wesentlichen roten Blutkörperchen und eine zweite Komponente aus Blutplättchen-reichem Plasma zu gewinnen, und Auftrennen des Blutplättchen-reichen Plasmas in eine dritte Komponente aus Blutplättchen-Konzentrat und in eine vierte Komponente aus praktisch Blutplättchen-freiem Plasma, dadurch gekennzeichnet, daß das Blutplättchen-reiche Plasma nicht durch Zentrifugieren in Blutplättchen-Konzentrat und praktisch Blutplättchen-freies Plasma getrennt wird, sondern durch Filtrieren des Blutplättchen-reichen Plasmas durch eine Mikroporen-Membran.

**Revendications**

1. Système fermé de collecte de composants sanguins comprenant des premiers moyens (14) pour recevoir du sang entier venant d'un donneur en vue de sa séparation en un premier composant constitué essentiellement de globules rouges et en un deuxième composant constitué de plasma riche en plaquettes, les premiers moyens (14) étant prévus pour l'utilisation avec un dispositif de traitement extra-corporel du sang par centrifugation (18) et comportant un récipient de centrifugation (26), utilisable avec le dispositif de traitement pour effectuer une centrifugation, et un premier conduit (28) raccorde au recipient de centrifugation pour introduire le sang entier venant d'un donneur dans ledit récipient de centrifugation pour sa séparation en globules rouges essentiellement et en plasma riche en plaquettes en réponse aux forces centrifuges; et des deuxièmes moyens (16) pour recevoir le plasma riche en plaquettes venant des premiers moyens et pour séparer le plasma en un troisième composant constitué de concentré de plaquettes et en un quatrième composant constitué de plasma pratiquement exempt de plaquettes, caractérisé en ce que les deuxièmes moyens (16) sont prévus pour la fixation à des moyens de pompage (24) et comprennent un dispositif à membrane microporeuse (22), pour séparer par filtration les composants cellulaires des composants non cellulaires du sang, et un deuxième conduit (42) qui connecte le récipient de centrifugation audit dispositif à membrane microporeuse et qui peut être fixé aux moyens de pompage pour introduire le plasma riche en plaquettes dans ledit dispositif à membrane microporeuse en vue de sa filtration en concentré de plaquettes et en plasma pratiquement exempt de plaquettes;

et le système comprend un premier récipient de transfert (74), un troisième conduit (76) qui communique avec ledit dispositif à membrane microporeuse et ledit premier récipient de transfert pour transférer ledit plasma pratiquement exempt de plaquettes dans ledit premier récipient de transfert, un deuxième récipient de transfert (78), et un quatrième conduit (80) qui communique avec ledit dispositif à membrane microporeuse et ledit deuxième récipient de transfert pour transférer ledit concentré de plaquettes dans ledit deuxième récipient de transfert; et chacun desdits conduits dans ledit système comprend une longueur de tube souple intégralement connectée avec ledit système pour former un chemin de fluide qui est fermé et ne communique pas avec l'atmosphère.

2. Système de collecte de composants du sang suivant la revendication 1, dans lequel chacun desdits récipients de centrifugation et des premier et deuxième récipients de transfert (26, 78, 74) comprend des moyens pour communiquer une caractéristique physique qui est favorable à la fonction de stockage prévue du système de collecte, telle que des caractéristiques améliorées de transmission des gaz pour une meilleure survie des plaquettes, une résistance mécanique relativement élevée à basse température pour faciliter la congélation du plasma pratiquement exempt de plaquettes, et la suppression de l'hémolyse dans les globules rouges.

3. Système de collecte de composants du sang suivant la revendication 1 ou 2, dans lequel lesdits premier moyens (14) comprennent un cinquième conduit (118) intégralement connecté avec ledit système et communiquant avec le récipient de centrifugation et servant à renvoyer les globules rouges au donneur.

4. Système de collecte de composants du sang suivant la revendication 3, dans lequel lesdits premiers moyens (14) comprennent un troisième récipient de transfert (124) et un sixième conduit (126) communiquant avec le conduit de retour (118) et le troisième récipient de transfert pour dériver un certain volume de globules rouges en dehors du donneur et le transférer dans le troisième récipient de transfert.

5. Système de collecte de composants du sang suivant l'une quelconque des revendications précédentes et comprenant en outre une source de solution anti-coagulante stérile (36) et une source (82) de solution saline stérile,

des moyens solidairement connectés audit système pour introduire la solution anti-coagulante dans ledit système,

et d'autres conduits (84) intégralement connectés audit système pour introduire la solution saline dans ledit système.

6. Procédé de séparation d'un volume de sang entier provenant d'un donneur, en composants, qui comprend la centrifugation du sang entier pour obtenir un premier composant constitué essentiellement de globules rouges et un deuxième composant constitué de plasma riche en plaquettes, et la séparation du plasma riche en plaquettes en un troisième composant constitué de concentré de plaquettes et en un quatrième composant constitué de plasma pratiquement exempt de plaquettes, caractérisé en ce que ledit plasma riche en plaquettes est séparé sans centrifugation en concentré de plaquettes et en plasma pratiquement exempt de plaquettes par filtration du plasma riche en plaquettes à travers une membrane microporeuse.

## FIG. 1

*FIG. 2*

*FIG. 3*

FIG. 4

FIG. 5

FIG. 6

FIG. 7

*FIG. 8*

*FIG. 9*

*FIG. 10*

*FIG. 11*